Europäisches Patentamt

European Patent Office

Office européen des brevets

(11) Publication number: **0 079 928**
**B1**

(12) **EUROPEAN PATENT SPECIFICATION**

(45) Date of publication of patent specification: **16.04.86**

(51) Int. Cl.⁴: **A 61 K 9/20**

(21) Application number: **82901785.4**

(22) Date of filing: **28.05.82**

(86) International application number:
**PCT/FI82/00019**

(87) International publication number:
**WO 82/04191 09.12.82 Gazette 82/29**

(54) **TABLETS WHICH DO NOT DAMAGE THE OESOPHAGUS.**

(30) Priority: **01.06.81 FI 811677**

(43) Date of publication of application:
**01.06.83 Bulletin 83/22**

(45) Publication of the grant of the patent:
**16.04.86 Bulletin 86/16**

(84) Designated Contracting States:
**AT BE CH DE FR GB LI NL SE**

(56) References cited:
**DE-A-2 217 132**
**DE-B-1 962 015**
**FI-A- 40 547**
**FR-A-2 210 399**
**FR-A-2 210 400**
**GB-A-1 380 741**

(73) Proprietor: **Orion-yhtymä Oy**
**Nilsiänkatu 10-14**
**SF-00510 Helsinki 51 (FI)**

(72) Inventor: **MARTTILA, Esko Veikko**
**Vespertie 3 C 25**
**SF-00320 Helsinki 32 (FI)**
Inventor: **SOTHMANN, Gunnar Aslak**
**Kaksosmäki 18**
**SF-02400 Kirkkonummi (FI)**
Inventor: **MARVOLA, Martti Lauri Antero**
**Elontie 24 as 3**
**SF-00660 Helsinki 66 (FI)**

(74) Representative: **Weinhold, Peter, Dr. et al**
**Patentanwälte Dr. V. Schmied-Kowarzik Dipl.-**
**Ing. G. Dannenberg Dr. P. Weinhold Dr. D. Gudel**
**Dipl.-Ing. S. Schubert Dr. P. Barz**
**Siegfriedstrasse 8**
**D-8000 München 40 (DE)**

Courier Press, Leamington Spa, England.

**Description**

The present invention is concerned with new tablet formulations prepared from emepronium bromide or tetracycline antibiotics such as doxycycline hydrochloride.

When drugs are administered orally in capsule or tablet form they can adhere to the oesophagus and damage it locally. Lately this problem has been studied with respect to tetracyclines such as doxycycline (see Med. J. Aust. 1975 1:236—7; JAMA 235 (1976) 2347—8; Läkartidningen 75 (1978) 49:4609—13).

Doxycycline hydrochloride is commercially available for oral use primarily as hard geletin capsules. When moistened in the mouth and throat the surface of the hard gelatin capsule becomes slimy and sticky if a large amount of liquid is not swallowed at the same time. If a capsule gets stuck in the oesophagus, swallowing large quantities of liquid will not loosen it. Thus the casing of the capsule and its contents dissolve in the oesophagus and may damage it locally. The damage caused by doxycycline hydrochloride arises primarily from its acidity.

Tablets do not tend to adhere to the oesophagus as easily as hard gelatin capsules. Doxycycline hydrochloride has thus also become commercially available in various tablet formulations. However tablets do not solve the problem completely, since various drugs have also caused damage by adhering in tablet form. The risk is especially great with slowly disintegrating tablets or non-disintegrating, long-acting matrix tablets.

It is coated tablets that usually stick (see Läkartidningen 75 (1978) 49:4613). The sticking mechanism is probably the same as in the case of hard gelatin capsules; i.e. the polymer of the film coating becomes slimy and sticky due to the action of fluids in the mouth and throat.

Surprisingly it has now been found that adherence to the oesophagus of tablets containing drugs can be reduced conciderably by adding a carbonate to the tablet which when moistened releases carbon dioxide due to the presence of the emebronium bromide or tetracycline antibiotics. Alkaline earth metal carbonates and bicarbonates are suitable though alkali metal carbonates and bicarbonates are preferred, sodium bicarbonate being the most preferred. The amount of carbonate can vary over a fairly wide range depending upon the drug, the tablet formulation and the desired neutralizing effect. A suitable amount would be from 0.01 to 3.0 equivalents of carbonate per equivalent of emebronium bromide or tetracycline antibiotics.

The tablets can be prepared by granulating the active agent with the usual auxiliary agents using a conventional wet or dry granulation method, then adding the carbonate to the dry granules and then tabletting the mixture.

It is also possible to add the carbonate at the granulation stage, but in that case the granulation must be carried out under completely dry conditions.

Emebronium bromide or tetracycline antibiotics which are not acidic enough to release carbon dioxide from carbonates can be made more acidic by adding a small amount of a pharmaceutically acceptable organic acid. The acid can be added by mixing the drug with the powdered acid or by moistening the drug with a solution of the acid followed by drying. Preparation is otherwise the same as for formulations containing emebronium bromide or tetracycline antibiotics. The organic acid are pharmaceutically acceptable acids, such as citric acid, tartaric acid and ascorbic acid. Suitable amounts are from 0.01 to 3.0 equivalents of acid per equivalent of carbonate.

The tablets can also be coated with a layer containing a carbonate which releases carbon dioxide slowly when moistened.

Sodium bicarbonate is used with an organic acid in effervescence tablets. The tablets disintegrate rapidly in water producing carbon dioxide and the solid tablet is thus transformed into a solution which can be taken as an effervescent drink. Effervescent tablets contain a substantially larger amount of carbonate and organic acid than the tablets described here.

It is believed that the reduced adherence of the new tablets described here to the oesophagus is due to small carbon dioxide bubbles formed on the surface of the tablet when it is moistened. The carbonate also neutralizes the surface of the tablet and its surroundings and thus reduces the local damaging effect of the emebronium bromide or tetracycline antibiotics. The amounts of carbonate are so small that they have no effect on the pH of the stomach or on the absorption of the drug.

The emebronium bromide or tetracycline antibiotics can also be made into a slowly disintegrating tablet or even into a non-disintegrating, long-acting matrix tablet without the danger of the tablet causing prolonged local stress by adhering to the oesophagus.

Doxycycline hydrochloride tablets prepared according to the formulations given here were compared with some commercially available preparations. Adherence was examined using a special device for measuring the force required to release a capsule which had adhered to the oesophagus of a pig. To compare the local stress caused by the acidity of the preparations one capsule or tablet was slurried in 10 ml of water and the pH of the slurry was measured.

The results of the comparison tests are presented in the following table.

TABLE

Comparison between some commercially available doxycycline hydrochloride preparations and preparations according to the formulations given here

| Preparation | Force required for release from oesophagus (N)±S.D. | pH of slurry in 10 ml of water |
|---|---|---|
| Hard gelatin capsule (100 mg) | 1.21±0.25 | 2.95 |
| Coated tablet I (150 mg) | 0.78±0.22 | 2.55 |
| Coated tablet II (100 mg) | 0.58±0.15 | 2.88 |
| Coated tablet III (100 mg) | 0.30±0.08 | 2.48 |
| Uncoated tablet (100 mg) | 0.29±0.07 | 2.55 |
| Tablet of Example I (100 mg) | 0.17±0.04 | 5.75 |
| Tablet of Example II (150 mg) | 0.16±0.03 | 6.35 |

Adherence to the oesophagus can be almost eliminated using the method presented here as the frictional force between the preparation and the oesophagus without real adherence is from 0.01 to 0.15 N. Additionally the pH values of the aqueous slurries of the preparations presented here are so high that adherence would probably not cause damage.

No differences in absorption were noticed between the preparations given here and commercially available preparations.

Among tetracycline antibiotics to which this invention can be applied are compounds such as doxycycline hydrochloride and tetracycline hydrochloride. Other agents belonging to the tetracycline group, such as oxytetracycline, chlortetracycline, demethylchlortetracycline and metacycline can also be prepared according to the formulations given here.

Emepronium bromide is one drug which damages the oesophagus, but is not sufficiently acidic to cause release of carbon dioxide from carbonates.

The following examples will further illustrate the invention.

Example 1

| | |
|---|---|
| Doxycycline hydrochloride respond. Doxycycline | 100 mg |
| Lactose (excipient) | 60 mg |
| Corn starch (excipient and disintegrant) | 10 mg |
| Polyvinyl pyrrolidone (binding agent) | 10 mg |
| Magnesium stearate (lubricant) | 2 mg |
| Sodium bicarbonate (adjuvant according to the invention) | 20 mg |

The active agent and excipient are granulated with the binding agent using a conventional wet or dry granulation method. The lubricant and adjuvant are blended with the dry granules and the mixture is compressed into tablettes.

Example 2

| | |
|---|---|
| Doxycycline hydrochloride respond doxycycline | 150 mg |
| Lactose (excipient) | 50 mg |
| Acrylic resin (binding agent) | 15 mg |
| Magnesium stearate (lubricant) | 3 mg |
| Sodium bicarbonate (adjuvant according to the invention) | 35 mg |

Tablets are prepared as in Example 1.

**0 079 928**

Example 3

| Tetracycline hydrochloride respond. tetracycline | 500 mg |
|---|---|
| Microcrystalline cellulose (excipient, binding agent) | 70 mg |
| Polyvinyl pyrrolidone (binding agent) | 15 mg |
| Silicon dioxide (glidant) | 2 mg |
| Magnesium stearate (lubricant) | 4 mg |
| Sodium bicarbonate (adjuvant according to the invention) | 70 mg |

Tablets are prepared as in Example 1.

Example 4

| Emepronium bromide | 100 mg |
|---|---|
| Citric acid (acidifying agent) | 6 mg |
| Lactose (excipient) | 100 mg |
| Hydrogenated castor oil (binding agent which removes the capping phenomenon) | 20 mg |
| Magnesium stearate | 2 mg |
| Calcium carbonate | 18 mg |

Tablets are prepared as in Example 1.

**Claims**

1. Oral tablets containing tetracycline antibiotics and a carbonate which releases carbon dioxide when the tablet is moistened in the mouth and throat.

2. Tablets according to claim 1, wherein doxycycline hydrochloride is used as the tetracycline antibiotic.

3. Tablets according to any one of the preceding claims, wherein alkali metal carbonate or bicarbonate is used as the carbonate.

4. Tablets according to claim 3, wherein sodium bicarbonate is used as the carbonate.

5. Tablets according to any one of the preceding claims, wherein 0.03—3.0 equivalents of the carbonate is used per equivalent of the drug.

6. Oral tablets containing emepronium bromide a carbonate and a sufficient amount of pharmaceutically acceptable organic acid to release carbon dioxide when the tablet is moistened in the mouth and throat.

7. Method for preparing tablets according to any one of the preceding claims, wherein the carbonate is added to the tablet composition at the granulation or tabletting stage or where the tablets are coated with a layer containing such a carbonate.

8. Method according to claim 7, wherein the carbonate is added at the granulation or tabletting stage.

9. Method according to claim 8, wherein the carbonate is blended with the dry granulation mixture before tabletting.

**Patentansprüche**

1. Orale, Tetracyclin-Antibiotika und ein Carbonat enthaltende Tabletten, die Kohlendioxid freisetzen, wenn die Tablette im Mund und Rachen befeuchtet wird.

2. Tabletten nach Anspruch 1, worin Doxycyclinhydrochlorid als Tetracyclin-Antibiotikum verwendet wird.

3. Tabletten nach einem der vorhergehenden Ansprüche, worin ein Alkalimetallcarbonat oder -bicarbonat als Carbonat verwendet wird.

4. Tabletten nach Anspruch 3, worin Natriumbicarbonat als Carbonat verwendet wird.

5. Tabletten nach einem der vorhergehenden Ansprüche, worin 0,03 bis 3,0 Äquivalente des Carbonates pro Äquivalent des Arzneimittels verwendet werden.

6. Orale Tabletten, die Emeproniumbromid, ein Carbonat und eine ausreichende Menge einer

4

**0 079 928**

pharmazeutisch annehmbaren organischen Säure zur Freisetzung von Kohlendioxid enthalten, wenn die Tablette im Mund und Rachen befeuchtet wird.

7. Verfahren zur Herstellung von Tabletten nach einem der vorhergehenden Ansprüche, worin das Carbonat der Tabletten-zusammensetzung in der Granulations- oder Tablettierungsstufe zugefügt wird oder bei dem die Tabletten mit einer ein derartiges Carbonat enthaltenden Schicht überzogen werden.

8. Verfahren nach Anspruch 7, worin das Carbonat in der Granulations- oder Tablettierungsstufe zugefügt wird.

9. Verfahren nach Anspruch 8, worin das Carbonat vor dem Tablettieren mit der trockenen Granulationsmischung gemischt wird.

**Revendications**

1. Comprimés à usage oral contenant des antibiotiques du type tétracycline et un carbonate qui libère du dioxyde de carbone lorsque le comprimé est humidifié dans la bouche et la gorge.

2. Comprimés selon la revendication 1, caractérisés en ce que le chlorhydrate de doxycycline est utilisé comme antibiotique du type tétracycline.

3. Comprimés selon l'une quelconque des revendications précédentes, caractérisés en ce que le carbonate utilisé est un carbonate ou un carbonate acide de métal alcalin.

4. Comprimés selon la revendication 3, caractérisés en ce que le carbonate utilisé est du carbonate acide de sodium.

5. Comprimés selon l'une quelconque des revendications 1 à 4, caractérisés en ce qu'on utilise 0,03 à 3,0 équivalents de carbonate par équivalent de médicament.

6. Comprimés à usage oral contenant du bromure d'emepronium, un carbonate et une quantité suffisante d'un acide organique acceptable du point de vue pharmaceutique pour libérer du dioxyde de carbone lorsque le comprimé est humidifié dans la bouche et dans la gorge.

7. Procédé de préparation de comprimés selon l'une quelconque des revendications 1 à 6, caractérisé en ce que le carbonate est ajouté à la composition du comprimé dans l'étape de granulation ou d'empastillage, ou que les comprimés sont revêtus d'une couche contenant ce carbonate.

8. Procédé selon la revendication 7, caractérisé en ce que le carbonate est ajouté lors de l'étape de granulation ou d'empastillage.

9. Procédé selon la revendication 8, caractérisé en ce que le carbonate est mélangé au mélange sec de granulation avant empastillage.

5